# EUROPEAN PATENT APPLICATION

(11) **EP 3 950 697 A1**
(43) Date of publication of application: **09.02.2022**
(21) Application number: 20784399.6
(22) Date of filing: 07.02.2020
(51) Int. Cl.: C07H 13/02, C12P 19/12, A61K 8/60

(54) **NOVEL COMPOUND AND USE OF SAME**

(30) Priority: 29.03.2019 JP 2019068164
(71) Applicant: Nagase & Co., Ltd., Osaka 550-8668 (JP); The Kitasato Institute, Tokyo 108-8641 (JP)
(72) Inventor: ZHANG, Wanjiao, Kobe-shi, Hyogo 651-2241 (JP); KOSAKA, Kunio, Kobe-shi, Hyogo 651-2241 (JP); MATSUYAMA, Keisuke, Kobe-shi, Hyogo 651-2241 (JP); TANAKA, Mami, Kobe-shi, Hyogo 651-2241 (JP); SOTA, Masahiro, Kobe-shi, Hyogo 651-2241 (JP)
(74) Representative: Vidon Brevets & Stratégie
(86) International application number: PCT/JP2020/004718
(87) International publication number: WO 2020/202790

(57) **Abstract**

It is an object of the present invention to provide a novel compound having an antioxidant effect and a technique of using the novel compound. The above object is achieved by providing a compound represented by the following formula (1): 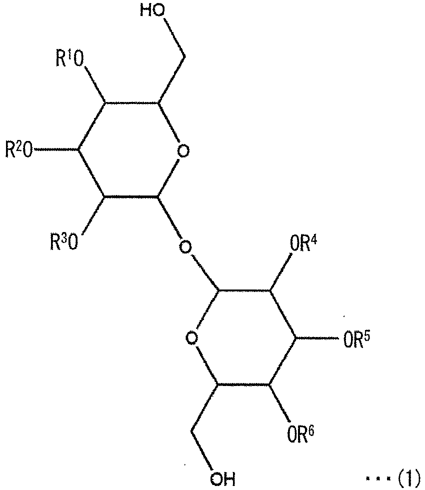 wherein any one of R¹ to R³ is hydrogen, an acetyl group, a 2-butenoyl group, or a 2-methyl-2-pentenoyl group, each of the other two of R¹ to R³ is hydrogen, any one of R⁴ to R⁶ is a 2-methyl-2-butenoyl group, and each of the other two of R⁴ to R⁶ is hydrogen.

## Description

### Technical Field

The present invention relates to a novel compound and a use of the novel compound. More specifically, the present invention relates to a novel compound, a composition containing the novel compound, a cosmetic composition or an antioxidant agent each containing the novel compound, and the like.

### Background Art

Metabolites produced by microorganisms are often useful substances for humans, and conventionally have been used as active components in medicines, cosmetics, etc.

For example, it is a famous story that penicillin having antibacterial activity was isolated from Penicillium. Further, as a recent example, it has been reported that a substance called trehangelin having functions such as an inhibitory effect on membrane lipid damage, an antibacterial activity, and an antioxidant effect was obtained from actinomycetes, as described in Patent Literatures 1 and 2.

Thus, there are many examples of obtaining useful substances from microorganisms, and searches for novel useful substances using microorganisms still have many potentialities.

### Citation List

### [Patent Literatures]

[Patent Literature 1]
   International Publication No. WO 2014/034147
[Patent Literature 2]
   Japanese Patent Application Publication Tokukai No. 2015-24985

### Summary of Invention

### Technical Problem

Under such circumstances, it is an object of an aspect of the present invention to provide a novel compound having an antioxidant effect and a technique of using the novel compound.

### Solution to Problem

As a result of diligent studies to achieve the above object, the inventors of the present invention succeeded in identifying a novel compound having an antioxidant effect from a specific microorganism, more specifically, an actinomycete *Streptomyces lividans* 1326 strain. In this way, the inventors accomplished the present invention. That is, an aspect of the present invention is a compound represented by the following formula (1): 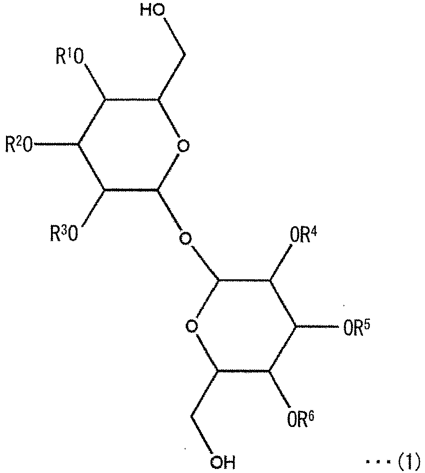 wherein any one of R¹ to R³ is hydrogen, an acetyl group, a 2-butenoyl group, or a 2-methyl-2-pentenoyl group, each of the other two of R¹ to R³ is hydrogen, any one of R⁴ to R⁶ is a 2-methyl-2-butenoyl group, and each of the other two of R⁴ to R⁶ is hydrogen.

### Advantageous Effects of Invention

According to an aspect of the present invention, it is possible to provide a novel compound having an antioxidant effect.

### Brief Description of Drawings

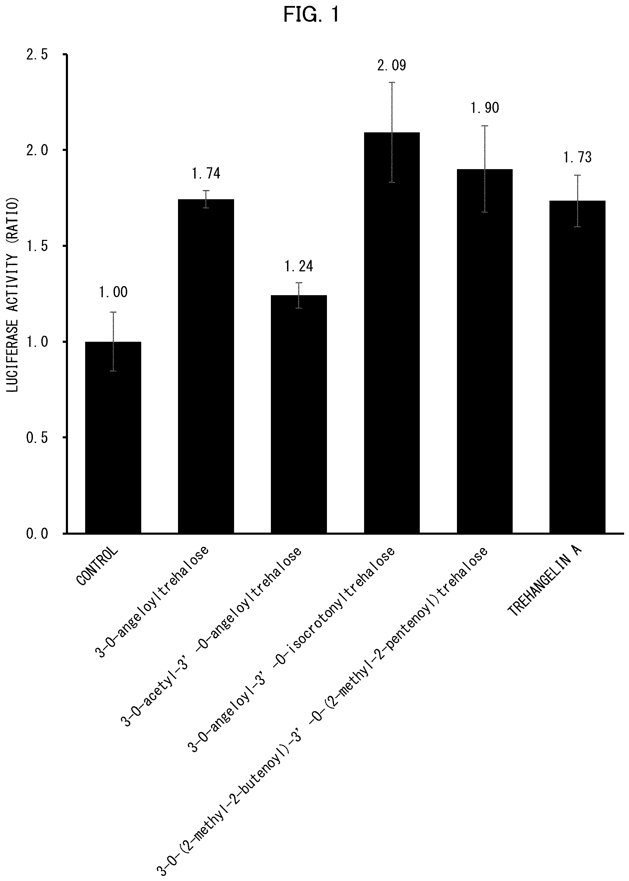

Fig. 1 is a graph showing the results of evaluation of an antioxidant effect of a compound in accordance with an aspect of the present invention.

### Description of Embodiments

The following description will discuss an embodiment of the present invention in detail.

Any numerical range expressed as "A to B" in the present specification means "not less than A and not more than B" unless otherwise stated. Further, in a case where the structural formulae in the present specification do not particularly represent a steric structure or the like, the compounds represented by the structural formulae in the present specification include various stereoisomers, such as tautomers, geometric isomers, and optical isomers, and mixtures thereof (including racemates).

### [1. Compound]

The inventors of the present invention conducted a detailed study on various microorganisms in order to search for a novel compound having an antioxidant effect. In the study, the inventors succeeded in identifying a novel compound having an antioxidant effect from an actinomycete *Streptomyces lividans* 1326 strain into which genes involved in a synthetic pathway of a specific compound were introduced.

A compound in accordance with an embodiment of the present invention (hereinafter referred to as "the present compound") is a compound represented by the above formula (1). The present compound has an excellent antioxidant effect as presented in Examples described later. Specific examples of such a compound will be discussed below. Note that the compound in accordance with the present invention may be in the form of a solvate, for example, a hydrate, and such a solvate is also encompassed in the scope of the present compound. Further, in the present specification, "2-butenoyl" is used in the same meaning as crotonyl and isocrotonyl, and "2-methyl-2-butenoyl" is used in the same meaning as angeloyl.

In an embodiment of the present invention, the present compound is preferably such that in the above formula (1), R² is hydrogen, an acetyl group, a 2-butenoyl group, or a 2-methyl-2-pentenoyl group, R⁵ is a 2-methyl-2-butenoyl group, and each of R¹, R³, R⁴ and R⁶ is hydrogen.

In the above preferable aspect, a compound in which R² in the above formula (1) is "hydrogen (H)" is 3-O-angeloyltrehalose, which is represented by the following formula (2):

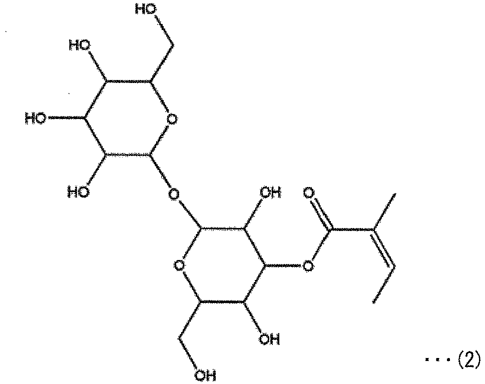

In the present specification, 3-O-angeloyltrehalose is a compound having the following physical characteristics:
(1) Properties: white powder
(2) Molecular weight: 424
(3) Molecular formula: C₁₇H₂₈O₁₂
(4) Melting point: 117°C to 120°C
(5) [M+NH₄]⁺ by high-resolution mass spectrometry theoretical value (m/z): 442.1925, measured value (m/z): 442.1901
(6) Ultraviolet absorption maximum (in methanol): 220 nm
(7) ¹H-NMR δ ppm: 1.86 (3H, dd, J₁ = 1.5 Hz, J₂ = 1.5 Hz), 1.93 (3H, dd, J₁ = 7.2 Hz, J₂ = 1.5 Hz), 3.15 (1H, ddd, J₁ = 9.5 Hz, J₂ = 9.1 Hz, J₃ = 5.7 Hz), 3.2-3.7 (9H, m), 3.78 (1H, ddd, J₁ = 10.0 Hz, J₂ = 4.0 Hz, J₃ = 2.3 Hz), 4.37 (1H, t, J=5.9Hz), 4.48 (1H, t, J=5.9 Hz), 4.72 (1H, d, J=6.4 Hz), 4.77 (1H, d, J = 5.6 Hz), 4.84 (1H, d, J = 5.7Hz), 4.84 (1H, d, J = 6.7 Hz), 4.91 (1H, d, J = 3.6 Hz), 4.95 (1H, d, J = 3.6 Hz), 5.02 (1H, d, J = 7.0 Hz), 5.21 (1H, t, J = 9.5 Hz), 6.04 (1H, dq, J₁ = 7.2 Hz, J₂ = 1.5 Hz)
(8) Solubility in solvent: Poorly soluble in methanol.

In the above preferable aspect, a compound in which R² in the above formula (1) is "an acetyl group ((CO)CH₃)" is 3-O-acetyl-3'-O-angeloyltrehalose, which is represented by the following formula (3):

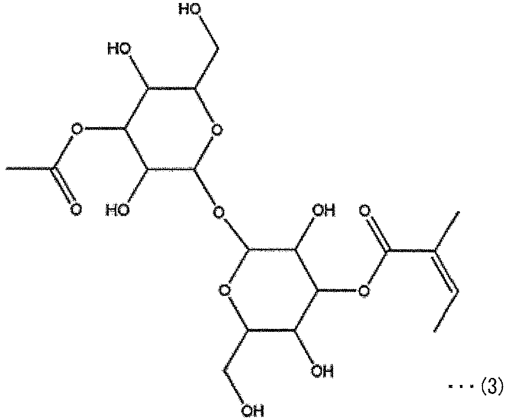

In the present specification, 3-O-acetyl-3'-O-angeloyltrehalose is a compound having the following physical characteristics:
(1) Properties: white powder
(2) Molecular weight: 466
(3) Molecular formula: C₁₉H₃₀O₁₃
(4) Melting point: 160°C to 161°C
(5) [M+NH₄]⁺ by high-resolution mass spectrometry theoretical value (m/z): 484.2030, measured value (m/z): 484.2001
(6) Ultraviolet absorption maximum (in methanol): 219nm
(7) ¹H-NMR δ ppm: 1.87 (3H, dd, J₁ = 1.4 Hz, J₂ = 1.4 Hz), 1.93 (3H, dd, J₁ = 7.1 Hz, J₂ = 1.4 Hz), 2.05 (3H, s), 3.2-3.5 (2H, m), 3.4-3.7 (6H, m), 3.7-3.8 (2H, m), 4.45 (1H, t, J = 5.6 Hz), 4.48 (1H, t, J = 5.6 Hz), 4.94 (1H, d, J = 7.3 Hz), 4.95 (1H, d, J = 6.7 Hz), 4.97 (2H, d, J = 3.7 Hz), 5.02 (1H, d, J = 6.2 Hz), 5.03 (1H, d, J = 7.0 Hz), 5.14 (1H, t, J = 9.5 Hz), 5.24 (1H, t, J = 9.5 Hz), 6.05 (1H, dq, J₁ = 7.1 Hz, J₂ = 1.4 Hz)
(8) Solubility in solvent: Poorly soluble in methanol.

In the above preferable aspect, a compound in which R² in the above formula (1) is "an isocrotonyl group ((CO)HC = CHCH₃))" is 3-O-angeloyl-3'-O-isocrotonyltrehalose, which is represented by the following formula (4):

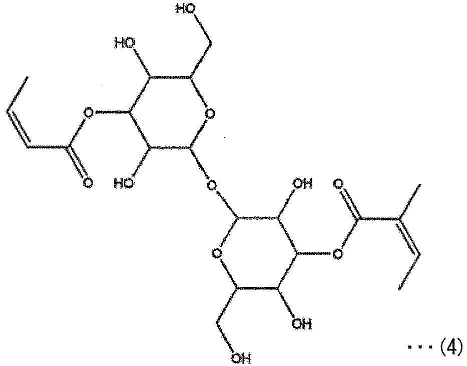

In the present specification, 3-O-angeloyl-3'-O-isocrotonyltrehalose is a compound having the following physical characteristics:
(1) Properties: white powder
(2) Molecular weight: 492
(3) Molecular formula: C₂₁H₃₂O₁₃
(4) Melting point: 155°C to 158°C
(5) [M+NH₄]⁺ by high-resolution mass spectrometry theoretical value (m/z): 510.2187, measured value (m/z): 510.2225
(6) Ultraviolet absorption maximum (in methanol): 227 nm
(7) ¹H-NMR δ ppm: 1.87 (3H, dd, J₁ = 1.4 Hz, J₂ = 1.4 Hz), 1.94 (3H, dd, J₁ = 7.1 Hz, J₂ = 1.4 Hz), 2.10 (3H, dd, J₁ = 7.2 Hz, J₂ = 1.7 Hz), 3.2-3.5 (2H, m), 3.4-3.7 (6H, m), 3.7-3.8 (2H, m), 4.46 (1H, t, J = 5.9 Hz), 4.48 (1H, t, J = 5.9 Hz), 4.92 (1H, d, J = 7.2 Hz), 4.93 (1H, d, J = 7.2 Hz), 4.97 (1H, d, J = 3.1 Hz), 4.98 (1H, d, J =3.1 Hz), 5.01 (1H, d, J = 7.3 Hz), 5.02 (1H, d, J = 7.4 Hz), 5.23 (1H, t, J = 9.5 Hz), 5.26 (1H, t, J = 9.5 Hz), 5.85 (1H, dd, J₁ = 11.5 Hz, J₂ = 1.7 Hz), 6.05 (1H, dq, J₁ = 7.1 Hz, J₂ = 1.4 Hz), 6.39 (1H, dq, J₁ = 11.5 Hz, J₂ = 7.2 Hz)
(8) Solubility in solvent: Poorly soluble in methanol.

In the above preferable aspect, a compound in which R² in the above formula (1) is "a 2-methyl-2-pentenoyl group ((CO)C(CH₃)=CHCH₂CH₃)" is 3-O-(2-methyl-2-butenoyl)-3'-O-(2-methyl-2-pentenoyl)treha lose, which is represented by the following formula (5):

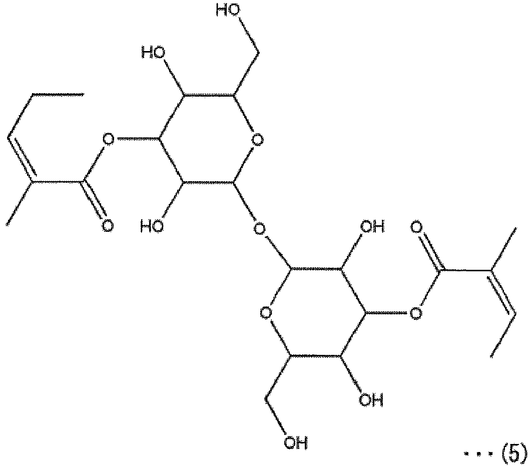

In the present specification, 3-O-(2-methyl-2-butenoyl)-3'-O-(2-methyl-2-pentenoyl)treha lose is a compound having the structure represented by the above formula (5) and having the following physical characteristics:
(1) Properties: white powder
(2) Molecular weight: 520
(3) Molecular formula: C₂₃H₃₆O₁₃
(4) Melting point: 176°C to 180°C
(5) [M+NH₄]⁺ by high-resolution mass spectrometry theoretical value (m/z): 538.2500, measured value (m/z): 538.2495
(6) Ultraviolet absorption maximum (in methanol): 227 nm
(7) ¹H-NMR δ ppm: 0.98 (3H, t, J = 7.5 Hz), 1.8-1.9 (6H, m), 1.94 (3H, dd, J₁ = 7.2 Hz, J₂ = 1.5 Hz), 2.41 (2H, ddq, J₁ = 7.5 Hz, J₂ = 7.5 Hz, J₃ = 1.1 Hz), 3.2-3.5 (2H, m), 3.4-3.7 (6H, m), 3.7-3.8 (2H, m), 4.48 (2H, t, J = 5.6 Hz), 4.917 (1H, d, J = 7.3 Hz), 4.925 (1H, d, J = 7.3 Hz), 4.98 (2H, d, J = 3.7 Hz), 5.01 (2H, d, J = 7.4 Hz), 5.26 (1H, t, J = 9.6 Hz), 5.27 (1H, t, J = 9.5 Hz), 5.93 (1H, dt, J₁ = 7.5 Hz, J₂ = 1.4 Hz), 6.05 (1H, dq, J₁ = 7.2 Hz, J₂ = 1.5 Hz)
(8) Solubility in solvent: Poorly soluble in methanol.

### [2. Composition]

A composition in accordance with an embodiment of the present invention (hereinafter referred to as "the present composition") contains at least one of the present compounds represented by the above formulae (1) to (5).

Further, in another embodiment, it is preferable that the present composition further contains at least one of the compounds represented by the following formulae (6) to (8). 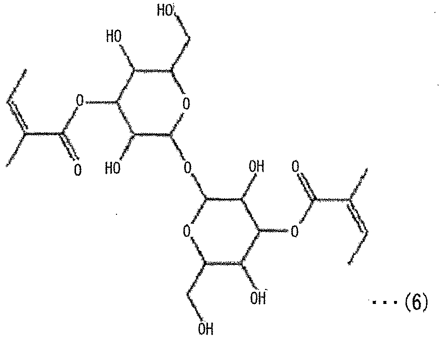 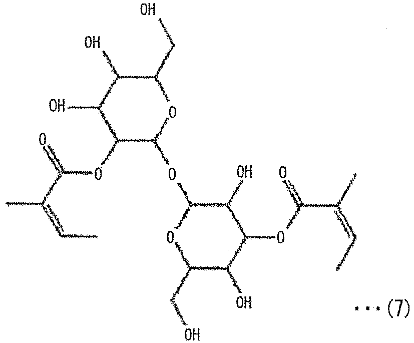 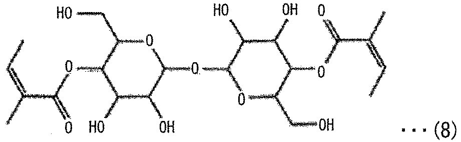 The compounds represented by the above formulae (6) to (8) are also referred to as "trehangelin A", "trehangelin B" and "trehangelin C", respectively. Further, in the present specification, the compounds represented by the above formulae (6) to (8) are collectively referred to as "trehangelin".

The present compound contained in the present composition has an antioxidant effect, and thus is useful in a variety of fields, in which an antioxidant substance is desired, of, for example, cosmetics, pharmaceuticals, foods, beverages, fragrances, pigments, synthetic resins, adhesives, fuels, and the like. Further, the compounds represented by the above formulae (6) to (8) have functions such as an inhibitory effect on membrane lipid damage, an antibacterial activity, and an antioxidant effect. Thus, by containing any of these compounds, the present composition can be expected to have a broader range of efficacy, a synergistic effect on the antioxidant effect, and the like.

The blending amount of the present compound contained in the present composition is in the order of 0.00001% by weight to 5% by weight as a preferable example, but the blending amount thereof can be set as appropriate according to various conditions such as a dosage form to be used and an intended use. In the above range, the blending amount of the present compound contained in the present composition is preferably 0.00001% by weight to 0.5% by weight, and more preferably 0.0001% by weight to 0.05% by weight.

Further, the blending amount of the compounds represented by the above formulae (6) to (8) contained in the present composition is in the order of 0.0001% by weight to 10% by weight as a preferable example, but the blending amount thereof can be set as appropriate according to various conditions such as a dosage form to be used and an intended use. In the above range, the blending amount of the present compound contained in the present composition is preferably 0.0001% by weight to 1% by weight, and more preferably 0.001% by weight to 0.1% by weight.

In the present composition, the blending ratio between the compound represented by the formula (1) of the present invention and trehangelin is, for example, 0.01:1 to 10:1, and preferably 0.4:1.

In the present composition, the blending ratio between the compound represented by the formula (2) of the present invention and trehangelin is, for example, 0.01:1 to 10:1, and preferably 0.2:1.

In the present composition, the blending ratio between the compound represented by the formula (3) of the present invention and trehangelin is, for example, 0.01:1 to 10:1, and preferably 0.04:1.

In the present composition, the blending ratio between the compound represented by the formula (4) of the present invention and trehangelin is, for example, 0.01:1 to 10:1, and preferably 0.05:1.

In the present composition, the blending ratio between the compound represented by the formula (5) of the present invention and trehangelin is, for example, 0.01:1 to 10:1, and preferably 0.05:1.

In the present composition, the blending ratio between the present compounds represented by the formulae (2) to (5) and trehangelin is, for example, 0.01:1 to 10:1, and preferably 0.34:1.

The present composition may contain not only trehangelin, but also various substances.

The following description will discuss a "cosmetic composition" and an "antioxidant agent" as an example of the present composition.

### [3. Cosmetic composition]

In an embodiment of the present invention, the present composition is preferably a cosmetic composition (hereinafter referred to as "the present cosmetic composition").

The present cosmetic composition preferably contains at least one of the compounds of the above formulae (1) to (5), and more preferably further contains any of the compounds represented by the above formulae (6) to (8).

The present cosmetic composition may contain, not only the above compounds, but also vegetable oil and other oils, higher fatty acids, higher alcohols, silicones, anionic surfactants, cationic surfactants, amphoteric surfactants, nonionic surfactants, preservatives, sugars, sequestrants, water-soluble polymers and other polymers, thickening agents, powder components, UV absorbents, UV blockers, hyaluronic acid and other moisturizing agents, aromatic agents, pH adjusters, drying agents, and the like. The present cosmetic composition may further contain: other medicinal components such as vitamins, skin activation agents, blood-circulation promoting agents, normal-bacteria controlling agents, active oxygen scavengers, anti-inflammatory agents, anticancer agents, whitening agents, and sterilizers; bioactive components; and the like.

Examples of the oils include: liquid oils such as camellia oil, evening primrose oil, macadamia nut oil, olive oil, rape seed oil, corn oil, sesame oil, jojoba oil, germ oil, wheat germ oil, triglycerol, and glycerin trioctanoate; solid oils such as cacao oil, coconut oil, hydrogenated coconut oil, palm oil, palm kernel oil, Japan wax, haze kernel oil, hydrogenated oil, and hydrogenated castor oil; waxes such as beeswax, candelilla wax, cotton wax, rice bran wax, lanolin, lanolin acetate, liquid lanolin, and sugarcane wax; liquid paraffin; squalene; squalane; microcrystalline wax; and the like.

Examples of the higher fatty acids include lauric acid, myristic acid, palmitic acid, stearic acid, oleic acid, linoleic acid, linolenic acid, docosahexaenoic acid (DHA), eicosapentaenoic acid (EPA), and the like.

Examples of the higher alcohols include: linear alcohols such as lauryl alcohol, stearyl alcohol, cetyl alcohol, and cetostearyl alcohol; branched alcohols such as monostearyl glycerin ether, lanolin alcohol, cholesterol, phytosterol, and octyldodecanol; and the like.

Examples of the silicones include: linear polysiloxanes such as dimethylpolysiloxane and methylphenylpolysiloxane; and cyclic polysiloxanes such as decamethyl polysiloxane.

Examples of the anionic surfactants include: fatty acid salts such as sodium laurate; higher alkyl sulfate salts such as sodium lauryl sulfate; alkyl ether sulfate salts such as POE lauryl sulfate triethanolamine salt; N-acyl sarcosinate; sulfosuccinic salt; and N-acyl amino acid salt and the like.

Examples of the cationic surfactants include: alkyl trimethyl ammonium salts such as stearyl trimethyl ammonium chloride; benzalkonium chloride; benzethonium chloride; and the like.

Examples of the amphoteric surfactants include: betaine surfactants such as alkyl betaine and amide betaine; and the like.

Examples of the nonionic surfactants include: sorbitan fatty acid esters such as sorbitan monooleate; and hardened castor oil derivatives.

Examples of the preservatives include methyl paraben, ethyl paraben, and the like.

Examples of the sequestrants include edetic acid, sodium edetate, and the like.

Examples of the polymers include: gum arabic, tragacanth gum, galactan, guar gum, carageenan, pectin, agar, quince seed, dextran, pullulan, carboxymethyl starch, collagen, casein, gelatin methyl cellulose, methylhydroxypropylcellulose, hydroxyethylcellulose, sodium carboxymethylcellulose (CMC), sodium arginate, vinyl-based polymers such as carboxyvinyl polymer (CARBOPOL, etc.), bentonite, and the like.

Examples of the thickening agents include carageenan, tragacanth gum, quince seed, casein, dextrin, gelatin, CMC, hydroxyethyl cellulose, hydroxypropyl cellulose, carboxyvinyl polymer, guar gum, xanthan gum, and the like.

Examples of the powder components include talc, kaolin, mica, silica, zeolite, polyethylene powder, polystyrene powder, cellulose powder, inorganic white pigment, inorganic red pigment, pearl pigments such as titanium-oxide coated mica, titanium-oxide coated talc, and colored titanium-oxide coated mica, organic pigments such as Red No. 201 and Red No. 202, and the like.

Examples of the UV absorbents include para-aminobenzoic acid, phenyl salicylate, isopropyl para-methoxycinnamate, octyl para-methoxycinnamate, 2,4-dihydroxybenzophenone, and the like.

Examples of the UV blockers include titanium oxide, talc, carmine, bentonite, kaolin, zinc oxide, and the like.

Examples of the moisturizing agents include polyethylene glycol, propylene glycol, dipropylene glycol, 1,3-butylene glycol, glycerin, diglycerin, polyglycerin, xylitol, maltitol, maltose, sorbitol, glucose, fructose, sodium chondroitin sulphate, sodium hyaluronate, sodium lactate, pyrrolidonecarboxylic acid, cyclodextrin, and the like.

Examples of the medicinal components include vitamins such as vitamin A oil, retinol and other vitamin As; riboflavin and other vitamin B2s; pyridoxine hydrochloride and other vitamin B6s; L-ascorbic acid, L-ascorbic acid phosphate ester, L-ascorbic acid monopalmitate, L-ascorbic acid dipalmitate, L-ascorbic acid-2-glucoside and other vitamin Cs; calcium pantothenate and other pantothenates; vimtain D2, cholecalciferol and other vitamin Ds; α-tocopherol, tocopherol acetate, DL-α-tocopherol nicotinate and other vitamin Es, and the like.

Other components that can be contained in the present cosmetic composition include: whitening agents such as placenta extract, glutathione, and Saxifraga sarmentosa extract; skin activation agents such as royal jelly and beech extract; blood-circulation promoting agents such as capsaicin, zingherone, cantharides tincture, ichthammol, caffeine, tannic acid, and γ-oryzanol; anti-inflammatory agents such as glycyrrhizic acid derivative, glycyrrhetinic acid derivative, and azulene; amino acids such as arginine, serine, leucine, and tryptophan; normal-bacteria controlling agents such as condensate of maltose and sucrose; lysozyme hydrochloride; and the like.

Still other substances that can be contained in the present cosmetic composition include various extracts such as chamomile extract, parsley extract, beech extract, wine yeast extract, grapefruit extract, woodbine extract, rice extract, grape extract, hop extract, rice bran extract, Eriobotrya japonica extract, phellodendron bark extract, coix seed extract, swertia herb extract, melilot extract, birch extract, glycyrrhiza extract, peony extract, Saponaria officinalis extract, Luffa cylindrica extract, capcicum extract, lemon extract, gentian extract, perilla extract, aloe extract, rosemary extract, sage extract, thyme extract, tea extract, seaweed extract, cucumber extract, clove extract, ginseng extract, Aesculus hippocastanum extract, Hamamelis virginiana extract, and Morus alba extract.

The present cosmetic composition can be applied in the form of, for example, a liquid preparation such as aqueous solution, oil, milky lotion, and suspension; a semi-solid preparation such as gel and cream; a solid preparation such as powder, granule, capsule, microcapsule, and solid. Preparation into these forms can carried out by conventionally known methods to provide a variety of dosage forms such as a lotion, emulsion, gel, cream, ointment, plaster, poultice, aerosol, suppository, injection, powder, granule, tablet, pill, syrup, and lozenge. These are applicable by application, plastering, spraying, drinking, and the like onto the body. In particular, among these dosage forms, dosage forms such as lotion, emulsion, cream, ointment, plaster, poultice, and aerosol are suitable for an external preparation for skin.

The present cosmetic composition can be prepared into a skin lotion, milky lotion, cream, pack and other skin care products; makeup base lotion, makeup cream, milky or creamy or paste foundation, lipstick, eye color, cheek color and other makeup products; hand cream, leg cream, body lotion and other body care products; bathing agents; oral care products; hair care products; and the like.

### [4. Antioxidant agent]

In an embodiment of the present invention, the present composition is preferably an antioxidant agent (hereinafter referred to as "the present antioxidant agent").

The present antioxidant agent can be formulated by a conventional method using a usual pharmaceutically acceptable carrier.

In the preparation of a solid preparation for oral administration, an excipient and, if necessary, an agent(s) such as a binder, a disintegrant, and a lubricant are added to a main medicine, and then the resulting mixture is prepared into a solvent, a granule, powder, a capsule, or the like by a conventional method.

In the preparation of an injection, if necessary, an agent(s) such as a pH adjuster, a buffer, a stabilizer, and a solubilizer is/are added to a main medicine, and then the resulting mixture is prepared into a subcutaneous or intravenous injection by a conventional method.

In the preparation of an external preparation for skin, the external preparation for skin can be prepared in line with the aforementioned present cosmetic composition. For example, the external preparation for skin can be an ointment preparation composed of (i) a hydrophobic or anhydrous solvent which is a mixture of one or more kinds of substances selected from the group consisting of fatty acid esters, higher alcohols, and propylene carbonate, all of which dissolve the present compounds represented by the above formulae (1) to (5), and (ii) a lipophilic base which is a mixture of one or more kinds of substances selected from white petrolatum, yellow petrolatum, liquid paraffin, and a polyethylene gel of liquid paraffin.

Further, a cream preparation can be a cream preparation containing: the present compound; an oil phase component composed of (i) a solid oil composed of 5 to 20 parts by weight of white petrolatum and 5 to 15 parts by weight of higher alcohols and (ii) a liquid oil composed of 3 to 10 parts by weight of squalane; an aqueous phase component; and 2.5 to 7.5 parts by weight of surfactants of two or more kinds. The oil phase component of the cream preparation may include other solid oil and other liquid oil, in addition to the above listed white petrolatum, higher alcohols, and squalane.

If necessary, an agent(s) such as a pH adjuster, a buffer, a stabilizer, and a solubilizer can be added to a main medicine, and then the resulting mixture is prepared into an external preparation including a semi-solid preparation such as an ointment and a cream, a liquid preparation such as a lotion, and a tape preparation by a conventional method.

The present antioxidant agent can be administered systemically or topically.

In the case where the present antioxidant agent is administrated systemically, the present antioxidant agent is administrated in dosage forms such as injections, oral preparations, and nasal preparations into blood vessels, tissues, gastrointestinal tracts, mucous membranes, and the like, and is administered in dosage forms such as aqueous injections, oily injections, tablets, granules, liquids, capsules, soft capsules, nasal drops, and nasal powders. The dose varies depending on the degree of symptoms, age, type of disease, and others, but is 50 mg to 500 mg per day for adults which is usually administered in a single dose or in two or more divided doses per day.

Further, in the case where the present antioxidant agent is administrated topically, the present antioxidant agent is administered directly to a diseased part of a skin in a dosage form which is an external preparation including a semi-solid preparation such as an ointment and a cream, a liquid preparation such as a lotion, and a tape preparation. The dose varies depending on the degree of symptoms, age, and type of disease, and others, but can be determined according to a common method of applying an anti-inflammatory external preparation for skin. For example, an appropriate amount of the external preparation may be applied once to several times per day according to a symptom, and can be applied several times according to the symptom.

### [5. Method for producing the present compound]

A method for producing the present compound in accordance with an embodiment of the present invention (hereinafter referred to as "the present production method") includes the steps of: (A) culturing, in a culture medium, a microorganism capable of producing the present compound represented by any of the above formulae (1) to (5); and (B) collecting the present compound represented by any of the above formulae (1) to (5) from the resulting culture.

The present compound can be produced by culturing, in a culture medium, a microorganism capable of producing the present compound, accumulating the present compound in the culture, and collecting (separating, extracting, and purifying) the present compound from the culture.

In the present production method, the "microorganism capable of producing the present compound" is not particularly limited, provided that it is a microorganism capable of producing the present compound. In addition, all bacteria capable of producing the present compound, such as mutant strains of the microorganism, genetically modified strains of the microorganism, and unknown wild strains that have not been identified, are included in the bacterial strains that can be used in the present production method.

Whether or not a microorganism is the "microorganism capable of producing the present compound" can be determined by culturing a test microorganism under conditions (culture temperature, pH, culture medium component, etc.) in which the test microorganism can grow appropriately, and then examining the presence or absence of the present compound in the culture. If the present compound is present in the culture, then it is possible to determine that the test microorganism is the microorganism capable of producing the present compound. Note that the conditions in which the above test microorganism can grow appropriately can be set as appropriate according to a microorganism to be cultured.

As used herein, the term "mutant strain" refers to a strain that is produced by artificial or natural mutagenesis stimulation and differs in mycological property or in gene from the microorganism capable of producing the present compound. Such a mutant strain includes bacterial strains derived from the microorganism capable of producing the present compound, and an original bacterial strain from which the microorganism capable of producing the present compound is derived. In the present specification, it does not matter whether any trace of actual derivation is left in the mutant strain. For example, a bacterial strain having a gene having high homology (for example, 80% or more, 85% or more, 90% or more, 95% or more, etc.) with a gene of the microorganism capable of producing the present compound (for example, 16S rRNA gene) is also included in the mutant strain. Further, it does not matter whether such a mutant strain is artificially produced or collected from nature, as long as the mutant strain maintains the capability of producing the present compound.

In the present specification, the "genetically modified strain" means a strain obtained by artificially introducing a gene into a wild strain of a microorganism from outside. The genetically modified strain is not particularly limited, provided that it has the capability of producing the present compound. The genetically modified strain is also referred to as a transformant.

A host of the genetically modified strain is not particularly limited. However, for example, a microorganism having no synthetic genes for the present compound on a genome can be used as the host of the genetically modified strain. In this case, the present compound can be produced by introducing the synthetic genes for the present compound into the microorganism.

Further, in another embodiment, a microorganism having synthetic genes for the present compound on a genome can be used as a host. Such a microorganism is capable of producing the present compound without the need to introduce any gene. However, introducing the synthetic genes for the present compound into such a microorganism has, for example, the advantage of increasing a production amount of the present compound. Examples of such a microorganism include *Polymorphospora rubra* K07-0510 strain (accession number NITE BP-01411) and the like. As discussed above, *Polymorphospora rubra* K07-0510 strain can produce the present compound by itself. Thus, *Polymorphospora rubra* K07-0510 strain into which any gene is not introduced is also encompassed in the "microorganisms capable of producing the present compound".

Examples of the host of the genetically modified strain include microorganisms and the like belonging to the following genera: *Escherichia*, *Corynebacterium*, *Brevibacterium*, *Bacillus*, *Microbacterium*, *Serratia*, *PSEudomonas*, *Agrobacterium*, *Alicyclobacillus*, *Anabaena*, *Anacystis*, *Arthrobacter*, *Azobacter*, *Chromatium*, *Erwinia*, *Methylobacterium*, *Phormidium*, *Rhodobacter*, *RhodopSEudomonas*, *Rhodospirillum*, *Scenedesmun*, *Streptomyces*, *Synnecoccus*, and *Zymomonas.* The host of the genetically modified strain is preferably microorganisms and the like belonging to the following genera: *Escherichia, Corynebacterium, Brevibacterium, Bacillus, PSEudomonas, Agrobacterium, Alicyclobacillus, Anabaena, Anacystis, Arthrobacter, Azobacter, Chromatium, Erwinia, Methylobacterium, Phormidium, Rhodobacter, RhodopSEudomonas, Rhodospirillum, Scenedesmun, Streptomyces, Synnecoccus,* and *Zymomonas.* Examples of actinomycetes include *Streptomyces albus, Streptomyces lividans, Streptomyces chromofuscus, Streptomyces exfoliatus, Streptomyces argenteorus,* and the like.

The host of the genetically modified strain is not particularly limited, provided that the host into which desired genes have been artificially introduced from outside is capable of producing the present compound. However, the host of the genetically modified strain is preferably actinomycetes, more preferably actinomycetes belonging to the genus *Streptomyces,* and particularly preferably *Streptomyces livedans.*

The production of the genetically modified strain is carried out by, for example, the method described in Japanese Patent Application Publication Tokukai No. 2017-158546. Briefly, a genetically modified strain (transformant) encompassed in the "microorganism capable of producing the present compound" is obtained by acquiring genes encoding the enzymes involved in synthesis of trehangelin (enoyl-CoA hydratase, 3-ketoacyl-CoA synthase, acyltransferase, and 3-ketoacyl-CoA reductase) on the basis of the description in Japanese Patent Application Publication Tokukai No. 2017-158546, and incorporating the acquired genes into a vector and introducing the vector into a host on the basis of the description in the same document.

That is, in another embodiment of the present invention, a method for producing the present compound includes: (C) a step of culturing, in a culture medium, a transformant (for example, an actinomycete) into which genes encoding enzymes involved in the synthesis of trehangelin have been introduced; and (D) a step of collecting the present compound represented by any of the above formulae (1) to (5) from the resulting culture.

In the present embodiment, the present compound is produced by culturing, in a culture medium, a transformant (for example, an actinomycete) into which a group of enzymes involved in the synthesis of trehangelin has been incorporated, accumulating the present compound in the resulting culture, and collecting (separating, extracting, and purifying) the present compound from the culture.

A culture medium for culturing the microorganism capable of producing the present compound may be any medium that can be used as a nutrient source for the microorganism. For example, nitrogen sources such as commercially available peptone, meat extract, corn steep liquor, cottonseed flour, peanut flour, soybean flour, yeast extract, NZ-amine, casein hydrate, sodium nitrate, ammonium nitrate, and ammonium sulfate, carbon sources including carbohydrates such as glycerin, starch, glucose, galactose, and mannose, and fats and the like, and inorganic salts such as sodium chloride, phosphate, calcium carbonate, and magnesium sulfate can be used alone or in combination. In addition, if necessary, a trace amount of metal salt, and oils serving as an antifoaming agent, such as animal oil, vegetable oil, and mineral oil, can be added. These culture media only need to be useful for the production of the present compound with use of production bacteria, and all known microorganism culture materials can be used.

Further, culturing of the microorganism capable of producing the present compound is carried out by culturing with shaking for several days to two weeks in a temperature range (for example, 10°C to 40°C, preferably 25°C to 30°C) which allows the production bacteria to grow and produce the present compound. The culture conditions can be appropriately selected and carried out according to the properties of production bacteria to be used for the present compound, with reference to the description in the present specification.

The collection of the present compound can be carried out by extracting it from a culture solution using a water-immiscible organic solvent such as ethyl acetate. In addition to this extraction method, known methods used for collection of fat-soluble substances, such as adsorption chromatography, partition chromatography, gel filtration chromatography, scraping from thin layer chromatography, centrifugal countercurrent partition chromatography, high performance liquid chromatography, can be combined appropriately or repeated to purify the present compound until it becomes pure.

The collection of the present compound can be carried out by, for example, the method described in Examples.

An embodiment of the present invention encompasses the following features:
<1> A compound represented by the following formula (1): 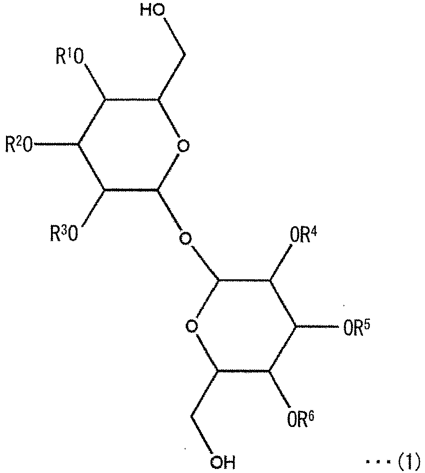 wherein any one of R¹ to R³ is hydrogen, an acetyl group, a 2-butenoyl group, or a 2-methyl-2-pentenoyl group, each of the other two of R¹ to R³ is hydrogen, any one of R⁴ to R⁶ is a 2-methyl-2-butenoyl group, and each of the other two of R⁴ to R⁶ is hydrogen.
<2> The compound described in <1>, wherein in the above formula (1), R² is hydrogen, an acetyl group, a 2-butenoyl group, or a 2-methyl-2-pentenoyl group, R⁵ is a 2-methyl-2-butenoyl group, and each of R¹, R³, R⁴ and R⁶ is hydrogen.
<3> A composition including a compound described in < 1 > or <2>.
<4> The composition described in <3>, further including at least one of compounds represented by the following formulae (6) to (8). 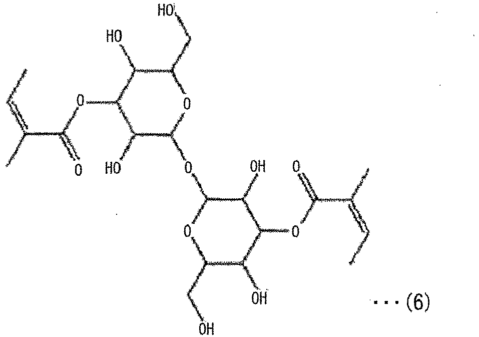 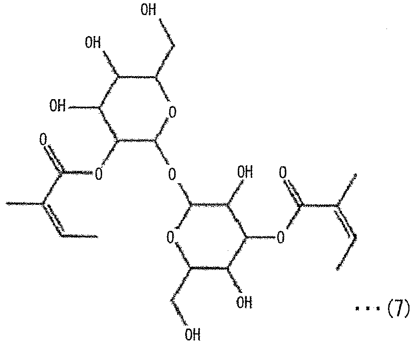 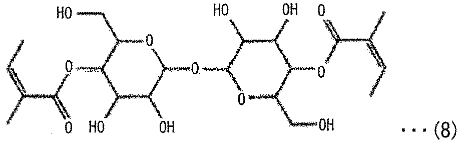
<5> The composition described in <3> or <4>, wherein the composition is a cosmetic composition or an antioxidant agent.
<6> A method for producing a compound described in < 1 > or <2>, including the steps of:
   (A) culturing, in a culture medium, a microorganism capable of producing the compound described in < 1 > or < 2 >; and
   (B) collecting the compound described in < 1 > or <2> from the resulting culture.

The present invention is not limited to the embodiments, but can be altered in various ways within the scope of the claims. The present invention also encompasses, in its technical scope, any embodiment derived by combining technical means disclosed in differing embodiments.

### Examples

The following description will more specifically describe the present invention with reference to Examples. However, the present invention is not limited to such Examples only.

### [1. Production of production bacterial strain]

An actinomycete *Polymorphospora rubra* K07-0510 strain was cultured in YD medium containing 1% yeast extract and 1% glucose at an appropriate temperature (for example, 27°C) for several days. After culturing, cells were obtained from the resulting culture solution by centrifugation. Then, chromosomal DNA was isolated and purified from the obtained cells according to a conventional method (Molecular Cloning, 2nd Edition).

For convenience, the four open reading frames (orfs) of SEQ ID NO: 1 were named orfA, orfB, orfC, and orfD in the ascending order of the base sequence numbers. The positions and functions of orfA to orfD in SEQ ID NO: 1 are as follows:
- orfA (SEQ ID NO: 2: base numbers 1 to 828 of SEQ ID NO: 1, encoding enoyl-CoA hydratase)
- orfB (SEQ ID NO: 3: base numbers 875 to 1900 of SEQ ID NO: 1, encoding 3-ketoacyl-CoA synthase)
- orfC (SEQ ID NO: 4: base numbers 1905 to 2684 of SEQ ID NO: 1, encoding acyltransferase)
- orfD (SEQ ID NO: 5: base numbers 2681 to 3475 of SEQ ID NO: 1, encoding 3-ketoacyl-CoA reductase)

A recombinant plasmid capable of sufficiently expressing the above four genes was constructed by PCR [Science, 230, 1350 (1985)] by the method described below.

A DNA capable of expressing orfA, orfB, orfC and orfD (hereinafter referred to as "orfABCD") was amplified by carrying out PCR on a DNA Thermal Cycler (manufactured by Applied Biosystems) with use of the chromosomal DNA of the actinomycete *Polymorphospora rubra* K07-0510 strain as a template, a sense primer of SEQ ID NO: 6 having a PstI restriction enzyme site and a ribosome binding sequence at the 5' terminus, an antisense primer of SEQ ID NO: 7 having a StuI restriction enzyme site at the 5' terminus, and Taq DNA polymerase (manufactured by Roche Life Science). The PCR was carried out under the conditions in which 30 cycles of a reaction step (1 cycle) consisting of 95°C for 30 seconds, 68°C for 30 seconds, and 72°C for 4 minutes were performed, followed by reaction at 72°C for 10 minutes. The amplified DNA fragment was purified by agarose gel electrophoresis, and digested with restriction enzymes PstI and StuI to obtain a DNA fragment (hereinafter referred to as "orfABCD-containing DNA fragment") containing a PstI and StuI-treated DNA capable of expressing orfABCD.

pOSV556t [Nat. Chem., 3, 338, (2011)] was digested with restriction enzymes PstI and StuI to obtain a PstI and StuI-treated pOSV556t fragment. The PstI and StuI-treated orfABCD-containing DNA fragment obtained above was mixed with the PstI and StuI-treated pOSV556t fragment, and a ligation reaction was then carried out to obtain a recombinant DNA.

The recombinant DNA was used to transform an E. *coli* Top 10 strain according to a conventional method, and the transformant was applied on a LB agar medium containing 100 µg/ml of ampicillin, and cultured overnight at 37°C. A plasmid containing the recombinant DNA was isolated from the transformant according to a conventional method. The recombinant DNA was sequenced to confirm that the recombinant DNA was orfABCD, and this plasmid was named pOSV556-orfABCD.

The pOSV556-orfABCD was introduced into E. *coli* ET12567/pUZ8002 strain [Practical Streptomyces Genetics (2000)] according to a conventional method to obtain E. *coli* ET12567/pUZ8002/pOSV556-orfABCD strain that is resistant to 50 µg/ml of kanamycin, 25 µg/ml of chloramphenicol, and 100 µg/ml of ampicillin. Further, the pOSV556-orfABCD was transferred by conjugation according to a conventional method from E. *coli* ET12567/pUZ8002 strain to an actinomycete *Streptomyces lividans* 1326 strain (the National Institute of Technology and Evaluation, Biotechnology Division, Biological Resource Center, NITE (NBRC): NBRC No. 15675) to obtain *Streptomyces* lividans/pOSV556-orfABCD that is resistant to 50 µg/ml of hygromycin.

### [2. Isolation and identification of novel compound]

The *Streptomyces* lividans/pOSV556-orfABCD obtained above was cultured with shaking at 27°C for 1 day in 500 ml of liquid medium containing 1% yeast extract and 1% glucose. Then, 50 ml of 20% trehalose aqueous solution was added, followed by further culturing with shaking at 27°C for 4 days. To the obtained culture solution was added 500 ml of ethanol, and the resulting mixture was stirred for 1 hour. Then, ethanol in an extract was removed under reduced pressure, 250 ml of ethyl acetate was added to the resulting aqueous solution. After a mixture was thoroughly stirred, an ethyl acetate phase was collected. Thereafter, the ethyl acetate phase was concentrated to dryness and dissolved in 100 mL of 0.1% formic acid.

An above obtained concentrated sample derived from 500 mL of the culture solution was used to purify the novel compound. The following columns and elution solvents were used for purification.

### <First purification>

- Column: ULTRA PACK ODS-SM-50B ∅26×300 mm (manufactured by YAMAZEN Corporation)
- Elution solvent: water/acetonitrile/formic acid (850/150/1)

### <Second purification>

- Column: through an open column, using 10g of Silica PSQ-100B
- Elution solvent: ethyl acetate

The purity of the fraction during purification was confirmed by HPLC. Samples with an LC purity of 95% or higher were collected and lyophilized to purify the novel compounds. Note that HPLC and LC/MS were carried out under the following analysis conditions.

### <HPLC analysis conditions>

- Device: Prominence UFLC system (manufactured by Shimadzu Corporation)
- Column: YMC-Pack ODS-AQ (manufactured by YMC Co., Ltd.)
- Mobile phase: water/acetonitrile/formic acid (850/150/1)
- Flow rate: 1 mL/min
- Detection: 220 nm

### <LC/MS analysis conditions>

- Device: Agilent Technologies 6224 TOF LC/MS (manufactured by Agilent Technologies)
- Column: YMC-Pack ODS-AQ (manufactured by YMC Co., Ltd.)
- Mobile phase: water/acetonitrile/formic acid (850/150/1)
- Flow rate: 0.5 mL/min

As a result, a novel compound A (25 mg), a novel compound B (12 mg), a novel compound C (15 mg), and a novel compound D (20 mg) were obtained. The following melting points and NMR analysis were performed on these compounds.

### (Results of melting point and NMR analysis)

The melting points and NMR of the above novel compounds A, B, C and D were measured under the following analysis conditions.

### <Melting point measurement conditions>

- Device: BUCHI Melting Point B-545 (manufactured by BUCHI)

### <NMR analysis conditions>

- Device: BRUKER AMX 400 (manufactured by Bruker)
- Solvent: DMSO-d6
- Internal standard: TMS

As a result, the novel compounds A, B, C and D were identified as 3-O-angeloyltrehalose, 3-O-acetyl-3'-O-angeloyltrehalose, 3-O-angeloyl-3'-O-isocrotonyltrehalose, and 3-O-(2-methyl-2-butenoyl)-3'-O-(2-methyl-2-pentenoyl)treha lose, respectively.

### [3. Physiological function evaluation]

The physiological functions of the compounds obtained above were evaluated. The evaluation of the physiological functions was carried out by using the tool described in Carnosic acid, a catechol-type electrophilic compound, protects neurons both in vitro and in vivo through activation of the Keap1/Nrf2 pathway via S-alkylation of targeted cysteines on Keap1. Takumi Satoh et al., Journal of Neurochemistry. Vol. 104, pp. 1116-1131, (2008).

Briefly, ARPE-19 cells were seeded in 24-well plates using a 10% FBS-DMEM/F12 medium (manufactured by GIBCO) so as to be 50% confluent. After 24 hours, (1) a mixed solution (vector solution) of 0.5 µg of pGL3-GSTYaARE-Luciferase vector, 0.5 µg of pSV-betaGAL vector, and 50 µL of Opti-MEM I (manufactured by GIBCO) per well and (2) a mixed solution (lipofectamine 2000) of 2 µL of lipofectamine 2000 (manufactured by Thermo Fisher) and 50 µL of Opti-MEM I (manufactured by GIBCO) per well were prepared and left at room temperature for 5 minutes. The vector solution and the lipofectamine 2000 solution were mixed and left at room temperature for 20 minutes. The resulting mixed solution was added to cultured cells (ARPE-19 cells) (100 µL per well) and cultured in a CO₂ incubator for 6 hours. Subsequently, the culture medium was replaced with a new 10% FBS-DMEM/F12 medium (0.5 mL/well), followed by culturing for 18 hours. The culture medium was then replaced with a new 10% FBS-DMEM/F12 medium (0.3 mL/well) or a 10% FBS-DMEM/F12 medium (0.3 mL/well) containing 20 mg/mL of various trehangelin derivatives, followed by culturing for 24 hours. After the medium was removed, 150 µL of PassiveLysisBuffer (manufactured by Promega) was added to prepare a cell lysis solution.

100 µL of each cell lysis solution was placed on a 96-well plate, and 100 µL of β-GAL assay kit solution (manufactured by Promega) was added, followed by incubation at 37°C for 3 hours. Then, β-GAL activity was confirmed by measuring the absorbance at 405 nm.

20 µL of each cell lysis solution was placed in a 96-well plate which differs from the above 96-well plate, and 100 µL of each luciferase assay kit solution (manufactured by Promega) was added. Then, luciferase activity was measured by measuring the luminescence. The obtained result on the luciferase activity was corrected by dividing them by the result on the β-GAL activity, and the antioxidant responsive element transcription activity was calculated. The results are shown in Fig. 1.

### (Results)

As can be seen from Fig. 1, 3-O-angeloyltrehalose, 3-O-acetyl-3'-O-angeloyltrehalose, 3-O-angeloyl-3'-O-isocrotonyltrehalose, and 3-O-(2-methyl-2-butenoyl)-3'-O-(2-methyl-2-pentenoyl)treha lose all showed the luciferase activity which was 1.24 to 2.09 times higher than that of a control. From this, 3-O-angeloyltrehalose, 3-O-acetyl-3'-O-angeloyltrehalose, 3-O-angeloyl-3'-O-isocrotonyltrehalose, and 3-O-(2-methyl-2-butenoyl)-3'-O-(2-methyl-2-pentenoyl)treha lose were all found to have an antioxidant effect.

Further, comparison with trehangelin A, which is a known substance, showed that 3-O-angeloyltrehalose has almost the same level of antioxidant effect as that of trehangelin A, and 3-O-angeloyl-3'-O-isocrotonyltrehalose and 3-O-(2-methyl-2-butenoyl)-3'-O-(2-methyl-2-pentenoyl)treha lose have a higher antioxidant effect than trehangelin A does.

### [4. Example formulation]

### (Skin lotion)

As a skin lotion containing a compound in accordance with an embodiment of the present invention, a skin lotion was produced with the following composition. At room temperature, the components (1) to (10) were added to the following component (11), and the resulting mixture was stirred. Then, the component (12) was added to the mixture and dissolved uniformly to obtain a lotion (unit: % by weight).

(1) Glycerin: 9.5
(2) 1,3-butylene glycol: 4.5
(3) Glucose: 1.5
(4) Ethanol: 5.0
(5) Carboxyvinyl polymer: 0.02
(6) Dipotassium glycyrrhizinate: 0.1
(7) Sodium hyaluronate: 0.1
(8) Compound in accordance with an embodiment of the present invention: 0.1
(9) Citric acid: 0.05
(10) Sodium citrate: 0.1
(11) Ion-exchanged water: remainder
(12) Potassium hydroxide: 0.01

### Industrial Applicability

The present invention is a novel compound having an antioxidant effect, and is therefore usable in the fields of, for example, cosmetics and pharmaceuticals.

## Claims

1. A compound represented by the following formula (1): 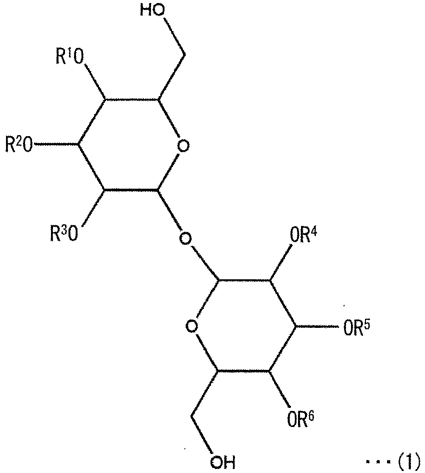 wherein any one of R¹ to R³ is hydrogen, an acetyl group, a 2-butenoyl group, or a 2-methyl-2-pentenoyl group, each of the other two of R¹ to R³ is hydrogen, any one of R⁴ to R⁶ is a 2-methyl-2-butenoyl group, and each of the other two of R⁴ to R⁶ is hydrogen.

2. The compound according to claim 1, wherein in the above formula (1), R² is hydrogen, an acetyl group, a 2-butenoyl group, or a 2-methyl-2-pentenoyl group, R⁵ is a 2-methyl-2-butenoyl group, and each of R¹, R³, R⁴ and R⁶ is hydrogen.

3. A composition comprising a compound according to claim 1 or 2.

4. The composition according to claim 3, further comprising at least one of compounds represented by the following formulae (6) to (8).

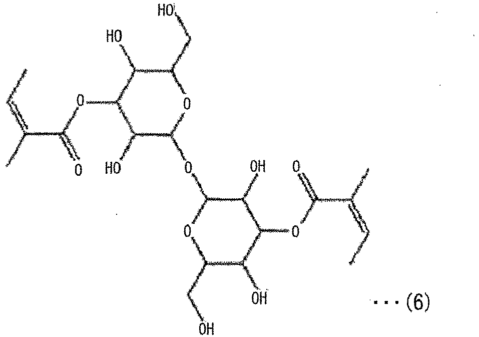

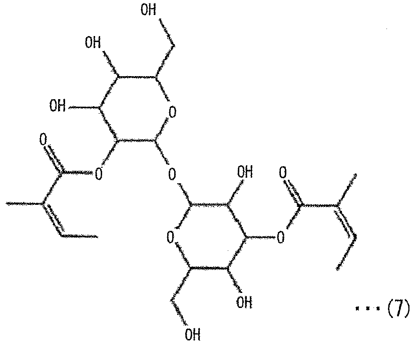

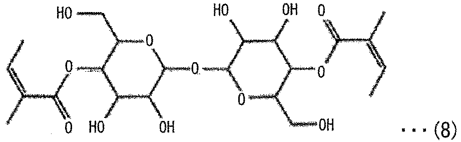

5. The composition according to claim 3 or 4, wherein said composition is a cosmetic composition or an antioxidant agent.

6. A method for producing a compound according to claim 1 or 2, comprising the steps of:
(A) culturing, in a culture medium, a microorganism capable of producing the compound according to claim 1 or 2; and
(B) collecting the compound according to claim 1 or 2 from the resulting culture.


SEQUENCE LISTING

<110>  NAGASE & CO., LTD.
       THE KITASATO INSTITUTE

<120>  Novel compound and use thereof

<130>  NGS19461

<150>  JP 2019-068164
<151>  2019-03-29

<160>  7

<170>  PatentIn version 3.5

<210>  1
<211>  3475
<212>  DNA
<213>  Polymorphospora rubra

<400>  1
gtgacccgac cggacgcccc gggcccaccg gtcgccgccc cggacccggc cgacgccgtc      60

gtgacggccg tcgagccgta cgtggtccgg gcgacgatca accgaccggc ccgccgcaac     120

gccatcgacc tcgccgtgat cgagggcctc gaacgcgcca tcgacctcgc cgaggcgacc     180

ggcgcccggg tcctggtgct gcgcggcgcc ggcggcacct tctgctccgg cgccgacctg     240

cgcgtcctgg aggagatgag cgtcgacccg caccgggtgg agacgttcat ggtgcggctc     300

gccctcgtgc tgcgccggct ggagaccgcc cggttcgtct cggtcgcggt cgtcgagggg     360

cacgccgtcg cgggcggctg cgagatcctg ctcgcctgcg acgtctcggt cgccgccacc     420

gacgcccgca tcggcgaccg ccatctcgag tacgggctgg tccccgccgc cggcggctcg     480

gtacggctgg cccgcaccct gcccaaggcc cggggcaact acctgctgct cgccgccgac     540

ctgctcaccg gggagcaggc ggcgcagtgg ggactggtca gcgtcgcggt gccgccaacc     600

gacctggaac cccgggtgga cgccctggtc gggcggctcg tcggccacag cgccgacgcg     660

ctcgccgtgg tcaagaagat ggtctggacc gccgaccacg aaccccggcc ggacgccatg     720

tcctgggaac gccggctctt cctgcgccac ctcggctccg aagacgtgtc cgaggggctg     780

cgcgccttcc gcgagcggcg ccggccggcg ttccgcgccg atgactgact ccgtacggcc     840

gcccgaccgg acggcccgac cgcaaggaga ccacatgtcg acctcgacgg tgatcggaac     900

aggttcgtac ctaccccgcc gcgtcctcag cagcggcgaa ctggcccgcc gggtcggtgt     960

ggcggagaac tggatcgtgg agaagaccgg gatccgggaa cgccgggtgg ccgccgacga    1020

ggaggccacc tccgacctgg ccacccgggc cgcgcgccgg gcgctgcgga ccgcccggct    1080

ggacccggcc gacgtcgacc tgatcgtgct ggcgacctcc acaccggacc ggccgatgcc 1140

ggccaccgcc agcaccgtgc aggccaacct cggcgcccgc caggcggtcg cgttcgacgt 1200

cgacgcggtg tgcagcggct tcgtgtacgc cctcgtcgtc gcccactcga tgctgaacag 1260

cgagggctgg gcccgtaccg cgctggtgat cggcgccgac acgtactcgc gggtcctgga 1320

ctacaccgac cggcgtaccg cggtgctctt cggcgacggt gccggcgcgg tcgtcctcgg 1380

ccgggagacc ggcggcggga gcggaatccg ggccaccacc ctgggcaccg acggcaccct 1440

cgccgacctc gtccagatcc cggccggcgg cagccgccgg ccggcgagcg cgcagacggt 1500

cgaggccggc gaccactact tcgcgatgcg cggcggcgac gtccgcaggc tggccaacca 1560

ggtcttcccg gcactggtcg ggcaactgct caaggcggcc tcgctcgacc tggaccaggt 1620

cgacctgatc gccgcccacc aggccaacgg cacgatgctc accgactggt cgcgggacct 1680

cggcctgcgc ccgggggtgc tgcaccgcac cgtcgagcgg tacggcaaca ccggtgccgc 1740

ctcggtcccg gtcaccctcg acgacgccgt acgcaccggg cggctcggtg ccgccgccac 1800

cctgctgatg gtcgccttcg gcggcgggat gacctggggc ggcgtcgccc tggactggtc 1860

cgccgacccg tcggttcccc gctccaactg cgtgaggtga agtgatgacc acgtccgcac 1920

tcggcgccga accgggcgcc accgatccgc cggccgcccc gccggtctcc tacgccccgt 1980

ccggccggat gccccggacg atcaaccgct ggccgcgccc gttcgcgccg cgcaacatcc 2040

tcggcgtgct gcgggcccgg ctgcgcggcg ccacgatcag cctcaaccag gtcaagttcg 2100

acgagtccac cgcggagcgc aacgcgctgg tgatggaaca cgcgctgctg cggcacagcc 2160

acgtcgcccg gtacgcgatc gtcggcccgt tcacctcgct gttcaaggtc cgggtcggcc 2220

cgtacgccgg gatcgccgag aaggtgaccg tcggtgccct gccgcactgg ccggaactgc 2280

cgaccagtca cgtcttcccg gtcaacgccg agttcggctt ctgcgcgggg gagtggccgg 2340

aggtgcccgg caccgaggtc ggcgccgacg cgtggatcgg cgccggcgcg gtggtccggg 2400

ccggcgtccg gatcgggcac ggcgcgatcg tcgcggccgg cgcggtcgtc acccgcgacg 2460

tcgccgacta cgagatcgtg gccggggtgc cggcccgccg gctgcggtcc cggttccccg 2520

acgacctggc cgaacggctg gtcgcgctgg cctggtggga ctggccgccg gggttcatca 2580

aggccaacat cgacctgttc cagcggccgc tgaccgccga caccctggcc gcgctggagg 2640

agcgggcccg cgccctgccc acacccccgg gtggtgccgc gtgaccgccg gtacggccca 2700

gccggtcgtc gccgcgcccg gcagggcgct cgccgaccgg gtggtggtgg tgaccggcgc 2760

cagccgcggt gtcggccgcg acctcgcccg ggtgttcgcc gaccacggcg cccgcctcgg 2820

cctgctggcc cgcagccggg acgcgctcga cgacctcggc ggcacgctca ccgccgccgg 2880

tgccgacgtc ctcgccgtgc cgtgcgacgt cggcgaaccg gactcgctgg ccggcgcggt 2940

cgacgccgtc gccgggcact tcggagggat cgactcggtc gtcgtcaacg ccggcatctc 3000

gcccgtcgcc cgtcgggcgc accacctgcc gatcgacgcc tggcacgacg tgctggcgac 3060

caacctgacc ggcgggttcg tcacggcgcg ggcggcgtat ccgcacctgg cccgctcggg 3120

gcgtggccgg ctggtgttca ccacctcggt catggccgcc accccgcggc gcgggctgag 3180

cgcgtacgcg gcgtcgaagg ccggcctgga ggggctcacc cgggccctgg ccgctgactg 3240

ggcgggggac ggaatcctgg tcaacgcggt cgcgccgggg ttcttcgaca ccgggctcgg 3300

cgccgccttc cacacctcgc agcgcctgca cgagcaggtc gtcggtcgta cgccggtggc 3360

ccggttcggc cgcgccgacg agttggccgc cgcgttcgtc ttcctggccg gtgacgcctg 3420

cggttacctg accggtcagg tgctcgccgt cgacggcggc tacggcctgg gctga 3475

<210> 2
<211> 828
<212> DNA
<213> Polymorphospora rubra

<400> 2
gtgacccgac cggacgcccc gggcccaccg gtcgccgccc cggacccggc cgacgccgtc 60

gtgacggccg tcgagccgta cgtggtccgg gcgacgatca accgaccggc ccgccgcaac 120

gccatcgacc tcgccgtgat cgagggcctc gaacgcgcca tcgacctcgc cgaggcgacc 180

ggcgcccggg tcctggtgct gcgcggcgcc ggcggcacct tctgctccgg cgccgacctg 240

cgcgtcctgg aggagatgag cgtcgacccg caccgggtgg agacgttcat ggtgcggctc 300

gccctcgtgc tgcgccggct ggagaccgcc cggttcgtct cggtcgcggt cgtcgagggg 360

cacgccgtcg cgggcggctg cgagatcctg ctcgcctgcg acgtctcggt cgccgccacc 420

gacgcccgca tcggcgaccg ccatctcgag tacggctggg tccccgccgc cggcggctcg 480

gtacggctgg cccgcaccct gcccaaggcc cggggcaact acctgctgct cgccgccgac 540

ctgctcaccg gggagcaggc ggcgcagtgg ggactggtca gcgtcgcggt gccgccaacc 600

gacctggaac cccgggtgga cgccctggtc gggcggctcg tcggccacag cgccgacgcg 660

ctcgccgtgg tcaagaagat ggtctggacc gccgaccacg aaccccggcc ggacgccatg 720

tcctgggaac gccggctctt cctgcgccac ctcggctccg aagacgtgtc cgaggggctg 780

cgcgccttcc gcgagcggcg ccggccggcg ttccgcgccg atgactga 828

<210>  3
<211>  1026
<212>  DNA
<213>  Polymorphospora rubra

<400>  3
atgtcgacct cgacggtgat cggaacaggt tcgtacctac cccgccgcgt cctcagcagc      60

ggcgaactgg cccgccgggt cggtgtggcg gagaactgga tcgtggagaa gaccgggatc     120

cgggaacgcc gggtggccgc cgacgaggag gccacctccg acctggccac ccgggccgcg     180

cgccgggcgc tgcggaccgc ccggctggac ccggccgacg tcgacctgat cgtgctggcg     240

acctccacac cggaccggcc gatgccggcc accgccagca ccgtgcaggc caacctcggc     300

gcccgccagg cggtcgcgtt cgacgtcgac gcggtgtgca gcggcttcgt gtacgccctc     360

gtcgtcgccc actcgatgct gaacagcgag ggctgggccc gtaccgcgct ggtgatcggc     420

gccgacacgt actcgcgggt cctggactac accgaccggc gtaccgcggt gctcttcggc     480

gacggtgccg gcgcggtcgt cctcggccgg gagaccggcg gcgggagcgg aatccgggcc     540

accaccctgg gcaccgacgg caccctcgcc gacctcgtcc agatcccggc cggcggcagc     600

cgccggccgg cgagcgcgca gacggtcgag gccggcgacc actacttcgc gatgcgcggc     660

ggcgacgtcc gcaggctggc caaccaggtc ttcccggcac tggtcggcca actgctcaag     720

gcggcctcgc tcgacctgga ccaggtcgac ctgatcgccg cccaccaggc caacggcacg     780

atgctcaccg actggtcgcg ggacctcggc ctgcgcccgg gggtgctgca ccgcaccgtc     840

gagcggtacg gcaacaccgg tgccgcctcg gtcccggtca ccctcgacga cgccgtacgc     900

accgggcggc tcggtgccgc cgccaccctg ctgatggtcg ccttcggcgg cgggatgacc     960

tggggcggcg tcgccctgga ctggtccgcc gacccgtcgg ttccccgctc caactgcgtg    1020

aggtga                                                               1026

<210>  4
<211>  780
<212>  DNA
<213>  Polymorphospora rubra

<400>  4
atgaccacgt ccgcactcgg cgccgaaccg ggcgccaccg atccgccggc cgccccgccg      60

gtctcctacg ccccgtccgg ccggatgccc cggacgatca accgctggcc gcgcccgttc     120

gcgccgcgca acatcctcgg cgtgctgcgg gcccggctgc gcggcgccac gatcagcctc     180

aaccaggtca agttcgacga gtccaccgcg gagcgcaacg cgctggtgat ggaacacgcg     240

ctgctgcggc acagccacgt cgcccggtac gcgatcgtcg gcccgttcac ctcgctgttc     300

aaggtccggg tcggcccgta cgccgggatc gccgagaagg tgaccgtcgg tgccctgccg 360

cactggccgg aactgccgac cagtcacgtc ttcccggtca acgccgagtt cggcttctgc 420

gcgggggagt ggccggaggt gcccggcacc gaggtcggcg ccgacgcgtg gatcggcgcc 480

ggcgcggtgg tccgggccgg cgtccggatc gggcacggcg cgatcgtcgc ggccggcgcg 540

gtcgtcaccc gcgacgtcgc cgactacgag atcgtggccg gggtgccggc ccgccggctg 600

cggtcccggt tccccgacga cctggccgaa cggctggtcg cgctggcctg gtgggactgg 660

ccgccggggt tcatcaaggc caacatcgac ctgttccagc ggccgctgac cgccgacacc 720

ctggccgcgc tggaggagcg ggcccgcgcc ctgcccacac ccccgggtgg tgccgcgtga 780

<210> 5
<211> 795
<212> DNA
<213> Polymorphospora rubra

<400> 5
gtgaccgccg gtacgggcca gccggtcgtc gccgcgcccg gcagggcgct cgccgaccgg 60

gtggtggtgg tgaccggcgc cagccgcggt gtcggccgcg acctcgcccg ggtgttcgcc 120

gaccacggcg cccgcctcgg cctgctggcc cgcagccggg acgcgctcga cgacctcggc 180

ggcacgctca ccgccgccgg tgccgacgtc ctcgccgtgc cgtgcgacgt cggcgaaccg 240

gactcgctgg ccggcgcggt cgacgccgtc gccgggcact tcggagggat cgactcggtc 300

gtcgtcaacg ccggcatctc gcccgtcgcc cgtcgggcgc accacctgcc gatcgacgcc 360

tggcacgacg tgctggcgac caacctgacc ggcgggttcg tcacggcgcg ggcggcgtat 420

ccgcacctgg cccgctcggg gcgtggccgg ctggtgttca ccacctcggt catggccgcc 480

accccgcggc gcgggctgag cgcgtacgcg gcgtcgaagg ccggcctgga ggggctcacc 540

cgggccctgg ccgctgactg ggcgggggac ggaatcctgg tcaacgcggt cgcgccgggg 600

ttcttcgaca ccgggctcgg cgccgccttc cacacctcgc agcgcctgca cgagcaggtc 660

gtcggtcgta cgccggtggc ccggttcggc cgcgccgacg agttggccgc cgcgttcgtc 720

ttcctggccg gtgacgcctg cggttacctg accggtcagg tgctcgccgt cgacggcggc 780

tacggcctgg gctga 795

<210> 6
<211> 37
<212> DNA
<213> Artificial Sequence

<220>
<223>  Primer

<400>  6
aaagggctgc agaggaggcg taccgtgacc cgaccgg                                37


<210>  7
<211>  30
<212>  DNA
<213>  Artificial Sequence

<220>
<223>  Primer

<400>  7
aaagggaggc ctgctcagcc caggccgtag                                        30